# EUROPEAN PATENT APPLICATION

(11) **EP 0 588 317 A1**
(43) Date of publication of application: **23.03.1994**
(21) Application number: 93114841.5
(22) Date of filing: 15.09.1993
(51) Int. Cl.: C07H 19/06, A61K 31/70

(54) **Uridine derivative and process for preparing the same**

(30) Priority: 17.09.1992 JP 247662/92
(71) Applicant: TANABE SEIYAKU CO., LTD., Chuo-ku Osaka (JP)
(72) Inventor: Tsujihara, Kenji, Urawa-shi, Saitama-ken (JP); Tanaka, Takashi, Urawa-shi, Saitama-ken (JP); Ohashi, Motoaki, Kawaguchi-shi, Saitama-ken (JP); Matsuda, Saburo, Nakagyo-ku, Kyoto-shi, Kyoto-fu (JP); Suzuki, Akira, Itami-shi, Hyogo-ken (JP)
(74) Representative: Hansen, Bernd, Dr. Dipl.-Chem.

(57) **Abstract**

A novel uridine derivative of the formula (I): 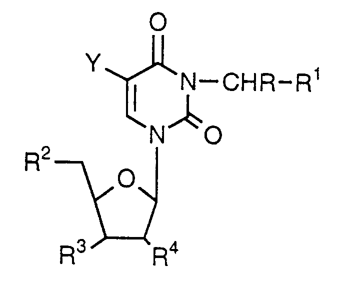
wherein R¹ is acyloxy, R² is hydrogen, acyloxy, etc., R³ is aralkyloxy, acyloxy, etc., R⁴ is hydrogen, acyloxy, etc., R is hydrogen or a lower alkyl, and Y is fluorine or trifluoromethyl, and a process for preparing the same are provided. The uridine derivative (I) of the present invention exhibits an excellent anti-tumor activity in vivo.

## Description

The present invention relates to a novel uridine derivative and a process for preparing the same. The uridine derivative of the present invention shows an excellent anti-tumor activity and low toxicity, therefore, is useful as an anti-tumor agent.

### Prior Art

2'-Deoxy-5-fluorouridine (hereinafter referred to as "FdUrd"), one of metabolites of 5-fluorouracil, is known to exhibit a cancer inhibitory activity. FdUrd is known to show an extremely strong activity to inhibit tumor growth. That is, it is reported that FdUrd has a cancer inhibitory activity more than 100 times higher than that of 5-fluorouracil at the same molar concentration in in vitro inhibition of growth of established cell line derived from mice.

However, FdUrd shows in vivo no advantage over 5-fluorouracil (Chemistry and Biology of Nucleosides and Nucleotides; Hanion, R.R.E., Robins, R.K., Townsend, L.B., Eds; Academic Press: New York, 1987; pp135-148). For this reason, FdUrd is currently used only in U.S.A. by arterial injection [cf. PHYSICIANS' DESK REFERENCE, 32nd ed., pp1387 (1976)].

In order to obtain an improved medicament, a variety of FdUrd derivatives have hitherto been explored, including, for example, 3-acyl-5-fluoro-2'-deoxyuridine (Japanese Patent First Publication 54-163586); 3',5'-diacyl-5-fluoro-2'-deoxyuridine (The Pharmaceutical Society of Japan, the 100th Conference, Summary pp321 (1980)); 5-fluoro-2'-deoxyuridine which is acylated at 3-position and both 3'- and 5'-positions (Japanese Patent First Publications 56-113795, 56-113796 and 56-113797); 3',5'-di-esters of FdUrd (Japanese Patent First Publication 58-49315); phosphoamidate derivative of FdUrd at 5'-position (Japanese Patent First Publication 57-128699); phosphate derivatives of FdUrd at 5'-position (Japanese Patent First Publications 58-99499, 59-70699, 59-93096, 61-91195, 61-91196, 61-152694 and 61-236793), and the like.

However, the various FdUrd derivatives as mentioned above are apt to show side effects on digestive organs such as diarrhea when orally administered. In addition, these derivatives are also disadvantageous in that they do not have a sufficient cancer inhibitory activity as compared to that of FdUrd and the dosage forms to be formulated are limited due to less water-solubility, e.g. they are hard to be formulated into injections.

### Summary of the Invention

Under the circumstances, the present inventors have intensively studied in order to solve the above-mentioned problems, and as a result, have found that the uridine derivative having a specific structure exhibits an excellent anti-tumor activity even in vivo. The uridine derivative also exhibits low toxicity.

The object of the present invention is to provide a uridine derivative of the following formula: 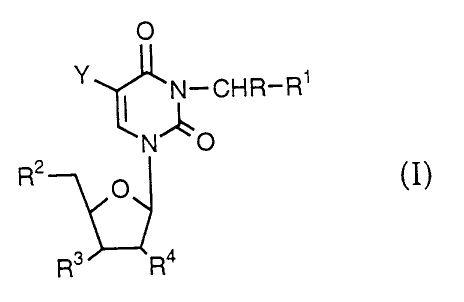
wherein R¹ is acyloxy; R² is hydrogen, phenoxycarbonyloxy (optionally substituted by a lower alkoxy), alkoxycarbonyloxy or acyloxy; R³ is aralkyloxy, phenoxycarbonyloxy (optionally substituted by a lower alkoxy), alkoxycarbonyloxy or acyloxy; R⁴ is hydrogen, phenoxycarbonyloxy (optionally substituted by a lower alkoxy), alkoxycarbonyloxy or acyloxy; R is hydrogen or a lower alkyl; and Y is fluorine or trifluoromethyl.

Another object of the invention is to provide a process for preparing said uridine derivative (I) of the invention.

These and other objects and advantages of the invention will be apparent to those skilled in the art from the following description.

### Detailed Description of the Invention

In the general formula (I), the acyl in the acyloxy group as R¹ includes a residue of a saturated or unsaturated aliphatic carboxylic acid having 4 to 20 carbon atoms, a lower alkanoyl group substituted by a lower alkoxycarbonyl, and the like, such as 2,2-dimethylpropionyl, butyryl, heptanoyl, octanoyl, palmitoyl, linolyl, a butyryl substituted by a lower alkoxycarbonyl, etc.

The acyl in the acyloxy group as R², R³ or R⁴ includes a straight chain or branched chain alkanoyl having 2 to 10 carbon atoms, a lower alkanoyl substituted by a lower alkoxycarbonyl, and the like, such as acetyl, propionyl, butyryl, isobutyryl, 2-(n-propyl)pentanoyl, heptanoyl, octanoyl, a propionyl substituted by a lower alkoxycarbonyl, a butyryl substituted by a lower alkoxycarbonyl, etc. The aralkyloxy group includes benzyloxy optionally substituted by a halogen atom. The examples of alkoxycarbonyloxy group are those having 2 to 20 carbon atoms, preferably 2 to 5 carbon atoms.

The lower alkyl and lower alkoxy used herein mean those having 1 to 6 carbon atoms, preferably 1 to 4 carbon atoms, and the lower alkanoyl means those having 2 to 6 carbon atoms, preferably 2 to 4 carbon atoms.

Among the compounds (I) of the present invention, preferred compounds in view of excellent pharmacological activities are the compounds (I) wherein R¹ is heptanoyloxy, both R² and R³ are butyryloxy or n-butoxycarbonyloxy, R⁴ is hydrogen, R is hydrogen and Y is fluorine.

The compounds (I) of the present invention include optical isomers due to the asymmetric carbon atom in the molecule, and these optical isomers and a mixture thereof are also included within the scope of the present invention.

The compounds (I) of the present invention are oily substance and are highly fat-soluble. The compounds (I) of the present invention may be used either in the form of a preparation suitable for oral administration such as capsules or in the form of a preparation suitable for parenteral administration (e.g. injections) such as fatty emulsions in view of its high solubility in soybean oil.

The dosage may vary depending on the administration route, age, weight and conditions of patients and the like, but is usually in the range of about 0.1 to 100 mg compound/kg body weight, preferably about 3 to 30 mg compound/kg body weight per day.

The uridine derivative of the formula (I) of the present invention can be prepared, for example, in accordance with the following reaction scheme: 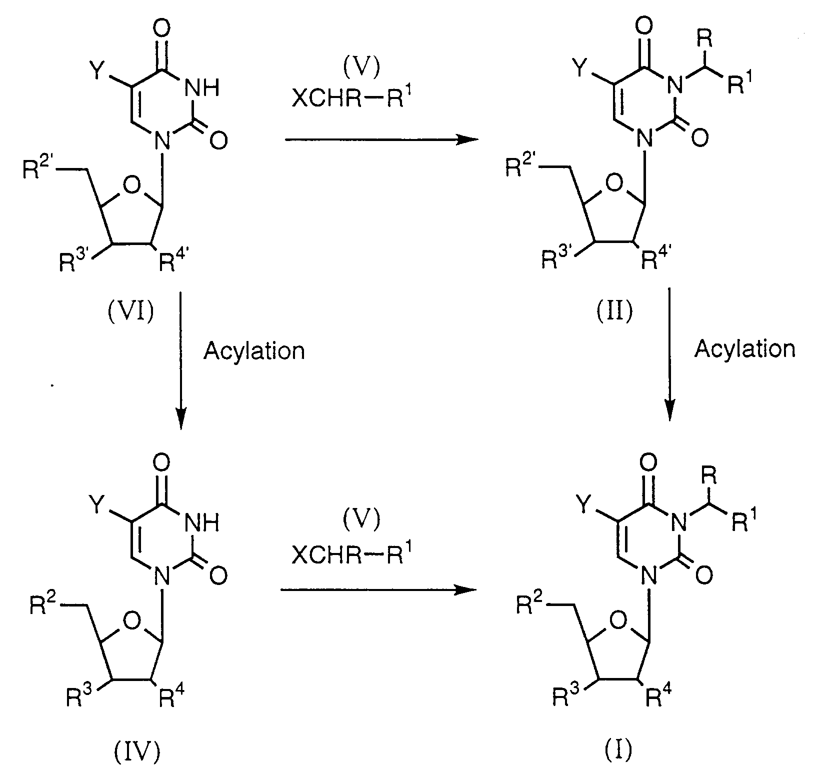
In the above reaction scheme, R¹, R², R³, R⁴, R and Y are as defined above, R^{2'} and R^{4'} is hydrogen or hydroxy, R^{3'} is hydroxy or aralkyloxy, X is a reactive residue.

That is, the starting uridine derivative of the formula (VI) is reacted with the compound (V) to produce the uridine derivative of the formula (II) wherein the CHRR¹ group at 3-position in the general formula (I) is introduced, followed by conventional acylation or oxycarbonylation (process 1); alternatively, the uridine derivative of the formula (VI) is first acylated or oxycarbonylated by a conventional method to produce the uridine derivative of the formula (IV), followed by reaction of the thus produced compound (IV) with the compound (V) to give the desired compound (I) (process 2).

The acylation reaction of the process 1 can be conducted by reacting the compound (II) with the compound of the general formula (III):

R⁵OH

wherein R⁵ is phenoxycarbonyl (optionally substituted by a lower alkoxy), alkoxycarbonyl or acyl, or a reactive derivative of the compound (III).

The condensation reaction of the compound (II) and the free carboxylic acid compound (III) can be carried out in the presence of a condensation agent. A suitable condensation agent includes, for example, N,N'-dicyclohexylcarbodiimide, N,N'-carbonyldiimidazole, 1-methyl-2-halopyridinium iodide, methoxyacetylene, triphenylphosphine tetrachloride, and the like. This reaction can proceed at a temperature between cooling and warming, for example, at 0 to 50°C.

The condensation reaction of the compound (II) and a reactive derivative of the compound (III) can be carried out in the presence or absence of an acid scavenger. The acid scavenger includes, for example, pyridine, tri(lower alkyl)amine, 4-dimethylaminopyridine, and the like. The reactive derivative of the carboxylic acid compound (III) may be any of those which can be used for peptide bond forming reaction, including, for example, an acid anhydride, a mixed acid anhydride (such as a mixed acid anhydride with carboxylic acid alkyl ester), acid halide, and the like. This reaction can proceed at a temperature between cooling and warming, for example, at 0 to 30°C.

The reaction of the compound (IV) and the compound (V) can be carried out in the presence or absence of an acid scavenger. The reactive residue can be, for example, a halogen atom, and the acid scavenger includes, for example, alkali metal hydrogen carbonate, alkali metal carbonate, pyridine, tri(lower alkyl)amine, 4-dimethylaminopyridine, and the like. This reaction can proceed at a temperature between cooling and warming, for example, at 0 to 30°C. Any of the condensation reactions as mentioned above can be conducted with or without a solvent. A suitable solvent is a conventional innert solvent, including, for example, acetone, methylene chloride, tetrahydrofuran, acetonitrile, and the like.

The reaction of the compound (VI) and the compound (V) can be conducted in the same manner as in the reaction of the compound (IV) and the compound (V), and the acylation reaction of the compound (VI) can be carried out in the same manner as in the reaction of the compound (II) with the compound (III) or a reactive derivative thereof.

In the above processes, the acyloxy group can be introduced simultaneously at R², R³ and R⁴, or in a stepwise manner in separate procedures so that different acyloxy groups are introduced into any of R², R³ and R⁴. For example, the acylation of the compound (VI) or (II) wherein both R^{2'} and R^{3'} are hydroxy and R^{4'} is hydrogen results in the formation of the corresponding compound wherein both R² and R³ are the same acyloxy group, the acylation of the compound wherein R^{2'} is hydrogen and both R^{3'} and R^{4'} are hydroxy results in the formation of the corresponding compound wherein both R³ and R⁴ are the same acyloxy group, and the acylation of the compound wherein R^{2'} is hydroxy, R^{3'} is aralkyloxy, and R^{4'} is hydrogen results in the formation of the corresponding compound wherein only R² is acyloxy.

The thus prepared uridine derivative (I) of the present invention shows an excellent anti-tumor activity and low toxicity, therefore, is useful for the treatment of various tumors in warm-blood animals including human beings. The compound of the present invention can be administered orally in the dosage form of tablets, capsules and powders, or parenterally in the dosage form of fat or lipid emulsions, if necessary, in admixture with pharmaceutically acceptable carrier, diluent or disintegrant. It is preferable to administer the compound parenterally as the fat emulsions (or lipid emulsions). That is, fat particulates present in fat emulsions (e.g. INTRALIPOS: trade name, manufactured by Midorijuji; INTRAFAT: trade name, manufactured by Nippon Seiyaku; etc.) used for the purpose of nutrient supplementation have also been used as a drug carrier like liposomes, and if the active compound of the present invention is administered with encapsulation into fat particulates (i.e. in the form of Lipid Microsphere (LM)), the pharmacological efficacy of the active compound is enhanced and the side effects are reduced as well.

The oil phase component used for preparing fat emulsions (or lipid emulsions) may be any of those conventionally used for preparing fat emulsions (or lipid emulsions), including vegetable oils such as soybean oil, cottonseed oil, synthetic triglycerides such as Panasate 800, Panasate 810 (trade name; manufactured by Nippon Yushi), squalene, eicosapentaenoic acid, azone, and the like. For preparing fat emulsions (or lipid emulsions), high solubility in these oil components is required for substance to be fat-emulsified (or lipid-emulsified). The compound of the present invention has a high solubility in these oil components, for example, 300 mg/ml or more in soybean oil at 40°C. The fat emulsions (or lipid emulsions) can be prepared by dissolving the compound of the present invention in soybean oil, Panasate, etc., adding lecithin to the solution, crude-emulsifying the mixture with a homomixer, etc., and then fine-emulsifying with a nanomizer under high pressure.

The compound of the present invention exhibits an excellent anti-tumor activity. For example, when fat emulsions containing the compound of the present invention, 3'-5'-O-di-n-butanoyl-3-n-heptanoyloxymethyl-3'-deoxy-5-fluorouridine, is intravenously administered to male mice inoculated with colon 26 cells in the inguinal region for 14 days (dose per day: 50 mg/kg), the growth inhibiting activity was shown by more than 90 % without decrease of body weight.

The present invention is explained in more detail by means of the following Examples but should not be construed to be limited thereto.

### Example 1

### Preparation of the compound (I) wherein R¹=CH₃(CH₂)₄-CH=CH-CH₂-CH=CH-(CH₂)₇COO (linolyloxy), R²=R³=CH₃(CH₂)₅COO, R⁴=H, R=H, Y=F

(a) 5-Fluoro-2'-deoxyuridine (1.53 g, 6.21 mmol) and sodium iodide (2.80 g, 18.7 mmol) were added to acetone (70 ml) and to the mixture were added chloromethyl linolate (3.07 g, 9.33 mmol) and anhydrous potassium carbonate (7 g) while stirring at room temperature. After the reaction was proceeded by stirring the mixture at room temperature overnight, the reaction product was concentrated under reduced pressure, ethyl acetate was added to the residue and insoluble materials were filtered off. After the filtrate was washed with water and then a saturated saline, it was dried and concentrated. The resulting residue (4.30 g) was purified with silica gel column chromatography (SiO₂: 150 g, solvent: ethyl acetate) to give 3-linolyloxymethyl-2'-deoxy-5-fluorouridine as colorless caramels (2.52 g) (yield: 75.3 %).
   IR(Neat): νₘₐₓ (cm⁻¹) 3460, 1730, 1670, 1470, 1270, 1100 FAB-MS (m/z): 539 (MH⁺)
(b) The product (1.50 g, 2.8 mmol) obtained in the above process (a) was dissolved in methylene chloride (30 ml) and thereto was added triethylamine (1.44 g, 14.3 mmol) while stirring under ice-cooling, and the mixture was added dropwise to a solution of n-heptanoic acid chloride in methylene chloride (1.41 g in 20 ml; 9.5 mmol). After reaction at room temperature for 2 hours, the reaction solution was washed with cold water, cold aqueous sodium hydrogen carbonate and saturated NaCl in this order. The solvent was distilled off and the resulting residue was purified with silica gel column chromatography (solvent: ethyl acetate/n-hexane=1/4, silica gel: 150 g) to give 3',5'-O-di-n-heptanoyl-3-linolyloxymethyl-2'-deoxy-5-fluorouridine as colorless oil (1.79 g) (yield: 83.7 %).
   IR(Neat): νₘₐₓ (cm⁻¹) 2960, 2930, 2850, 1740, 1695, 1680
   FAB-MS (m/z): 763 (MH⁺)

### Example 2

### Preparation of the compound (I) wherein R¹=CH₃(CH₂)₄-CH=CH-CH₂-CH=CH-(CH₂)₇COO (linolyloxy), R²=R³=n-BuOCO(CH₂)₂COO, R⁴=H, R=H, Y=F

The procedures of Example 1(b) were repeated except that the product obtained in Example 1(a) (2.3 g, 4.3 mmol) and succinic acid mono-n-butyl ester chloride (2.23 g, 13 mmol) were used to give 3',5'-O-di-n-butoxycarbonylpropionyl-3-linolyloxymethyl-2'-deoxy-5-fluorouridine (2.77 g) (yield: 75.9 %).
IR(Neat): νₘₐₓ (cm⁻¹) 3100, 3000, 2970, 2920, 2850, 1740, 1700, 1680
FAB-MS (m/z): 851 (MH⁺)

### Example 3

### Preparation of the compound (I) wherein R¹=CH₃(CH₂)₄-CH=CH-CH₂-CH=CH-(CH₂)₇COO (linolyloxy), R²=R³=CH₃COO, R⁴=H, R=H, Y=F

The procedures of Example 1(b) were repeated except that the product obtained in Example 1(a) (540 mg, 1.00 mmol) and anhydrous acetic acid (0.5 ml, 5 mmol) were used to give 3',5'-O-diacetyl-3-linolyloxymethyl-2'-deoxy-5-fluorouridine (616 mg) as colorless oil (yield: 98.9 %). The solubility of this compound in soybean oil was >300 mg/ml.
IR(Neat): νₘₐₓ (cm⁻¹) 1740, 1680, 1470, 1360, 1280, 1230, 1110
FAB-MS (m/z): 645 (M+Na)⁺

### Example 4

### Preparation of the compound (I) wherein R¹=CH₃(CH₂)₅COO, R²=R³=CH₃(CH₂)₂COO, R⁴=H, R=H, Y=F

(a) The procedures of Example 1(a) were repeated except that 5-fluoro-2'-deoxyuridine (4.95 g, 20.1 mmol), anhydrous potassium carbonate (15 g), sodium iodide (12 g, 80 mmol) and chloromethyl n-heptanoate (7.19 g, 40.2 mmol) were used to give 3-n-heptanoyloxymethyl-2'-deoxy-5-fluorouridine as colorless solid (4.77 g) (yield: 61.1 %) (melting point: 104 to 106°C).
(b) The procedures of Example 1(b) were repeated except that the product obtained in the above process (a) (583 mg, 1.50 mmol), butyryl chloride (480 mg, 4.50 mmol, 1.5 equivalents) and triethylamine (1.05 ml, 7.5 mmol, 5 molar equivalents) were used to give 3',5'-O-dibutyryl-3-n-heptanoyloxymethyl-2'-deoxy-5-fluorouridine as colorless oil (706 mg) (yield: 89.0 %). The solubility of this compound in soybean oil was >300 mg/ml.
   IR(Neat): νₘₐₓ (cm⁻¹) 1740, 1690, 1680, 1470, 1280, 1170, 1100
   FAB-MS (m/z): 529 (MH⁺)
   ¹H-NMR (CDCl₃) δ: 0.88 (3H, t, J=7 Hz), 0.97 (6H, t, J=7 Hz), 1.28 (6H, m), 1.60-1.73 (6H, m), 2.17 (1H, ddd, J=14, 8, 7 Hz), 2.29-2.39 (6H, m), 2.55 (1H, ddd, J=14, 6, 3Hz), 4.28 (1H, m), 4.32, 4.44 (1H each, dd, J=12, 3 Hz), 5.22 (1H, ddd, J=6, 3, 3 Hz), 5.98 (2H, s), 6.31 (1H, ddd, J=8, 6, 2 Hz), 7.71 (1H, d, J=6 Hz)

### Example 5

### Preparation of the compound (I) wherein R¹=CH₃(CH₂)₅COO, R²=R³=(CH₃)₂CHCOO, R⁴=H, R=H, Y=F

The procedures of Example 1(b) were repeated except that the product obtained in Example 4(a) (1.2 g, 3.09 mmol), isobutyryl chloride (990 mg) and triethylamine (2.2 ml) were used to give 3',5'-O-diisobutyryl-3-n-heptanoyloxymethyl-2'-deoxy-5-fluorouridine (1.313 g) (yield: 80.4 %).
IR(Neat): νₘₐₓ (cm⁻¹) 2980, 2940, 1740, 1695, 1680
FAB-MS (m/z): 529 (MH⁺)

### Example 6

### Preparation of the compound (I) wherein R¹=CH₃(CH₂)₅COO, R²=R³=(CH₃CH₂CH₂)₂CHCOO, R⁴=H, R=H, Y=F

The procedures of Example 1(b) were repeated except that the product obtained in Example 4(a) (1.1 g, 2.8 mmol), 2-propylpentanoyl chloride (1.64 g) and triethylamine (2.4 ml) were used to give 3',5'-O-di-(2-propylpentanoyl)-3-n-heptanoyloxymethyl-2'-deoxy -5-fluorouridine (1.126 g) (yield: 63.0 %).
IR(Neat): νₘₐₓ (cm⁻¹) 2970, 2930, 1740, 1695, 1680
FAB-MS (m/z): 663 (MH⁺)

### Example 7

### Preparation of the compound (I) wherein R¹=CH₃(CH₂)₅COO, R²=R³=CH₃(CH₂)₅COO, R⁴=H, R=H, Y=F

The procedures of Example 1(b) were repeated except that the product obtained in Example 4(a) (1.1 g, 2.8 mmol), 2-heptanoyl chloride (1.66 g) and triethylamine (2.4 ml) were used to give 3',5'-O-di-n-heptanoyl-3-n-heptanoyloxymethyl-2'-deoxy-5-fluorouridine (1.56 g) (yield: 91.1 %).
IR(Neat): νₘₐₓ (cm⁻¹) 2970, 2740, 1740, 1695, 1680
FAB-MS (m/z): 635 (M+Na)⁺

### Example 8

### Preparation of the compound (I) wherein R¹=CH₃(CH₂)₅COO, R²=R³=n-BuOCO(CH₂)₂COO, R⁴=H, R=H, Y=F

The procedures of Example 1(b) were repeated except that the product obtained in Example 4(a) (1 g, 2.6 mmol), succinic acid mono-n-butyl ester chloride (1.80 g) and triethylamine (2.2 ml) were used to give 3',5'-O-di-n-butoxycarbonylpropiony-3-n-heptanoyloxymethyl-2'-deoxy-5-fluorouridine (1.58 g) (yield: 87.0 %).
IR(Neat): νₘₐₓ (cm⁻¹) 2970, 2940, 1740, 1695, 1680
FAB-MS (m/z): 723 (M+Na)⁺

### Example 9

### Preparation of the compound (I) wherein R¹=CH₃(CH₂)₅COO, R²=R³=n-BuOCO(CH₂)₃COO, R⁴=H, R=H, Y=F

The procedures of Example 1(b) were repeated except that the product obtained in Example 4(a) (564 mg), glutaric acid mono-n-butyl ester chloride (1.19 g) and triethylamine (1.2 ml) were used to give 3',5'-O-di-n-butoxycarbonylbutyryl-3-n-heptanoyloxymethyl-2'-deoxy-5-fluorouridine as yellow oil (1.04 g) (yield: 98.4 %).
IR(Neat): νₘₐₓ (cm⁻¹) 3100, 2970, 2930, 2870, 1740, 1690, 1680
FAB-MS (m/z): 751 (M+Na)⁺

### Example 10

### Preparation of the compound (I) wherein R¹=CH₃(CH₂)₁₄COO (palmityloxy), R²=R³=(CH₃)₂CHCOO, R⁴=H, R=H, Y=F

(a) The procedures of Example 1(a) were repeated except that 5-fluoro-2'-deoxyuridine (4.5 g, 18.3 mmol), anhydrous potassium carbonate (13.66 g, 98.8 mmol), sodium iodide (10.11 g, 67.4 mmol) and chloromethyl palmitate (11.15 g, 2 equivalents) were used to give 3-palmityloxymethyl-2'-deoxy-5-fluorouridine (6.39 g) (yield: 67.8 %). This product was further purified by recrystallization from ethyl acetate/hexane to give a pure colorless product (6.17 g) (melting point: 87 to 89°C).
(b) The procedures of Example 1(b) were repeated except that the product obtained in the above process (a) (1.6 g, 3.1 mmol), triethylamine (2.2 ml) and isobutyryl chloride (991 mg) were used to give 3',5'-O-diisobutyryl-3-palmityloxymethyl-2'-deoxy-5-fluorouridine as red oil (2.116 g) (yield: 104.2 %). This product was further purified by silica gel column chromatography (solvent: ethyl acetate/hexane=1:3) to give a pure product (2.06 g).
   IR(Neat): νₘₐₓ (cm⁻¹) 2930, 2840, 1740, 1695, 1680
   FAB-MS (m/z): 655 (MH⁺)

### Example 11

### Preparation of the compound (I) wherein R¹=CH₃(CH₂)₁₄COO (palmityloxy), R²=R³=n-CH₃(CH₂)₅COO, R⁴=H, R=H, Y=F

The procedures of Example 1(b) were repeated except that the product obtained in Example 10(a) (1.3 g, 2.5 mmol), n-heptanoyl chloride (1.11 g) and triethylamine (1.8 ml) were used to give 3',5'-O-di-n-heptanoyl-3-palmityloxymethyl-2'-deoxy-5-fluorouridine (1.194 g) (yield: 64.6 %).
IR(Neat): νₘₐₓ (cm⁻¹) 2970, 2930, 2850, 1745, 1700, 1680
FAB-MS (m/z): 739 (MH⁺)

### Example 12

### Preparation of the compound (I) wherein R¹=CH₃(CH₂)₁₄COO (palmityloxy), R²=R³=n-BuOCO(CH₂)₂COO, R⁴=H, R=H, Y=F

The procedures of Example 1(b) were repeated except that the product obtained in Example 10(a) (1.8 g, 3.5 mmol) and succinic acid mono-n-butyl ester chloride (1.83 g) were used to give 3',5'-O-di-n-butoxycarbonylpropionyl-3-palmitiloxymethyl-2'-deoxy-5-fluorouridine as oil (1.94 g) (yield: 67.0 %).
IR(Neat): νₘₐₓ (cm⁻¹) 2960, 2930, 2850, 1740, 1695, 1680
FAB-MS (m/z): 827 (MH⁺)

### Example 13

### Preparation of the compound (I) wherein R¹=n-BuOCO(CH₂)₃COO R²=R³=CH₃(CH₂)₅COO, R⁴=H, R=H, Y=F

(a) The procedures of Example 1(a) were repeated except that 5-fluoro-2'-deoxyuridine (3.2 g, 13 mmol), anhydrous potassium carbonate (9.70 g, 70 mmol), sodium iodide (7.11 g, 47 mmol) and glutaric acid n-butyl and chloromethyl ester (6.2 g, 26 mmol) were used to give 3-n-butoxybutyryloxymethyl-2'-deoxy-5-fluorouridine (5.35 g) (yield: 92.2 %).
   IR(Neat): νₘₐₓ (cm⁻¹) 3500, 2970, 2930, 2880, 1735
   FAB-MS (m/z): 447 (MH⁺)
(b) The procedures of Example 1(b) were repeated except that the product obtained in the above process (a) (300 mg, 0.7 mmol), triethylamine (0.5 ml) and n-heptanoyl chloride (320 mg) were used to give 3',5'-O-di-n-heptanoyl-3-n-butoxybutyryloxymethyl-2'-deoxy-5-fluorouridine (203 mg) (yield: 45.0 %) .
   IR(Neat): νₘₐₓ (cm⁻¹) 2970, 2930, 2850, 1740, 1695, 1680
   FAB-MS (m/z): 671 (MH⁺)

### Example 14

### Preparation of the compound (I) wherein R¹=t-BuCOO (pivaloyloxy), R²=R³=CH₃(CH₂)₅CHCOO, R⁴=H, R=H, Y=F

(a) The procedures of Example 1(a) were repeated except that 5-fluoro-2'-deoxyuridine (2.5 g, 10.2 mmol), anhydrous potassium carbonate (7.59 g), sodium iodide (5.53 g) and chloromethyl pivalate (3.07 g, 2 equivalents) were used to give 3-pivaloyloxymethyl-2'-deoxy-5-fluorouridine as oil (2.96 g) (yield: 80.5 %).
   IR(Neat): νₘₐₓ (cm⁻¹) 3480, 3080, 2980, 2930, 1735, 1670
   FAB-MS (m/z): 361 (MH⁺)
(b) The procedures of Example 1(b) were repeated except that the product obtained in the above process (a) (1.1 g, 3.1 mmol), triethylamine (2.2 ml) and n-heptanoyl chloride (1.38 g) were used to give 3',5'-O-di-n-heptanoyl-3-pivaloyloxymethyl-2'-deoxy-5-fluorouridine as oil (1.49 g) (yield: 82.2 %).
   IR(Neat): νₘₐₓ (cm⁻¹) 2960, 2930, 1740, 1695, 1680
   FAB-MS (m/z): 585 (MH⁺)

### Example 15

### Preparation of the compound (I) wherein R¹=CH₃(CH₂)₄-CH=CH-CH₂-CH=CH-(CH₂)₇COO (linolyloxy), R²=R³=phenoxycarbonyloxy, R⁴=H, R=H, Y=F

The procedures of Example 1(b) were repeated except that the product obtained in Example 1(a) (2.05 g, 3.80 mmol) and phenyl chloroformate (1.9 ml, 15.1 mmol, about 2 equivalents) were used to give 3',5'-O-diphenoxycarbonyl-3-linolyloxymethyl-2'-deoxy-5-fluorouridine (2.81 g) (yield: 94.8 %). The solubility of this compound in soybean was 150 mg/0.5 ml.
IR(Neat): νₘₐₓ (cm⁻¹) 2930, 1760, 1740 (sh), 1690, 1680, 1250, 1070
FAB-MS (m/z): 779 (MH⁺)

### Example 16

### Preparation of the compound (I) wherein R¹=CH₃(CH₂)₄-CH=CH-CH₂-CH=CH-(CH₂)₇COO (linolyloxy), R²=R³=4-methoxyphenoxycarbonyloxy, R⁴=H, R=H, Y=F

The procedures of Example 1(b) were repeated except that the product obtained in Example 1(a) (2.05 g, 3.80 mmol) and 4-methoxyphenyl chloroformate (about 1 mole/L when the purity is estimated at 100 %) (20 ml) were used to give 3',5'-O-di(4-methoxyphenoxy-carbonyl)-3-linolyloxymethyl-2'-deoxy-5- fluorouridine (3.10 g) (yield: 97.2 %). The solubility of this compound in soybean was 150 mg/0.5 ml.
IR(Neat): νₘₐₓ (cm⁻¹) 1760, 1740 (sh), 1690, 1680, 1510, 1240, 1210
FAB-MS (m/z): 839 (MH⁺)

### Example 17

### Preparation of the compound (I) wherein R¹=CH₃(CH₂)₅COO, R²=R³=n-butoxycarbonyloxy, R⁴=H, R=H, Y=F

The procedures of Example 1(b) were repeated except that the product obtained in Example 4(a) (2.25 g, 5.79 mmol) and n-butyl chloroformate (3.48 g, 25.5 mmol) were used to give 3',5'-O-di-n-butoxycarbonyl-3-n-heptanoyloxymethyl-2'-deoxy-5-fluorouridine as colorless oil (2.63 g) (yield: 77.2 %).
IR(Neat): νₘₐₓ (cm⁻¹) 1750, 1690, 1680, 1470, 1260
FAB-MS (m/z): 589 (MH⁺)
¹H-NMR (CDCl₃) δ: 0.88 (3H, t, J=7 Hz), 0.95 (3H, t, J=7 Hz), 0.96 (3H, t, J=7 Hz), 1.25-1.48 (10H, m), 1.61-1.73 (6H, m), 2.24 (1H, ddd, J=15, 9, 7 Hz), 2.32 (2H, t, J=7 Hz), 2.60 (1H, ddd, J=14, 6, 2 Hz), 4.18 (4H, m), 4.35 (1H, m), 4.43 (2H, d, J= 3 Hz), 5.19 (1H, ddd, J= 6, 2, 2 Hz), 5.97 (2H, s), 6.42 (1H, ddd, J=9, 6, 2 Hz), 7.73 (1H, d, J=6 Hz)

### Example 18

### Preparation of the compound (I) wherein R¹=CH₃(CH₂)₄-CH=CH-CH₂-CH=CH-(CH₂)₇COO (linolyloxy), R²=n-butyryloxy, R³=4-chlorobenzyloxy, R⁴=H, R=H, Y=F

(a) The procedures of Example 1(a) were repeated except that 3'-o-(4-chlorobenzyl)-2'-deoxy-5-fluorouridine (1.92 g, 5.17 mmol), chloromethyl linolate (2.90 g, 8.82 mmol), anhydrous potassium carbonate (7 g) and sodium iodide (2.65 g, 17.7 mmol) were used to give 3'-O-(4-chlorobenzyl)-3-linolyloxymethyl-2'-deoxy-5-fluorouridine (2.27 g) (yield: 66.2 %).
   IR(Nujol): νₘₐₓ (cm⁻¹) 3520, 1730, 1715, 1665, 1465, 1270, 1120, 1100, 1045, 760
   FAB-MS (m/z): 663 (MH⁺)
(b) The procedures of Example 1(b) were repeated except that the product obtained in the above process (a) (2.23 g, 3.36 mmol), n-butyryl chloride (717 mg, 6.72 mmol) and triethylamine (2.3 ml, 16.5 mmol) were used to give 3'-O-(4-chlorobenzyl)-5'-O-n-butyryl-3-linolyloxymethyl-2'-deoxy-5-fluorouridine as colorless caramel (2.39 g) (yield: 97.0 %).
   IR(Nujol): νₘₐₓ (cm⁻¹) 1740, 1670, 1460, 1380, 1270, 1180, 1100, 1080
   FAB-MS (m/z): 733 (MH⁺)

### Example 19

### Preparation of the compound (I) wherein R¹=CH₃(CH₂)₄-CH=CH-CH₂-CH=CH-(CH₂)₇COO (linolyloxy), R²=R³=R⁴=n-butyryloxy, R=H, Y=F

(a) 5-Fluorouridine (2.0 g, 7.62 mmol) was dissolved in pyridine (60 ml) and thereto was added dropwise a solution of n-butyryl chloride in tetrahydrofuran (2.52 g in 10 ml; 11.8 mmol). After the mixture was stirred at room temperature for 2 hours, the reaction solution was concentrated under reduced pressure. To the residue were added ethyl acetate and water, the mixture was stirred, and the organic layer was separated. After the organic layer was washed with 5 % aqueous HCl solution, saturated NaHCO₃ and saturated NaCl, it was dried and concentrated. The obtained residue (3.42 g) was purified by silica gel chromatography (solvent: ethyl acetate/n-hexane) to give 2',3',5'-O-tri-n-butyryl-5-fluorouridine as colorless caramel (3.52 g) (yield: 70.0 %).
   IR(Nujol): νₘₐₓ (cm⁻¹) 3240, 3100, 1750, 1730, 1710, 1675, 1460, 1380, 1170
   FAB-MS (m/z): 473 (MH⁺)
(b) The procedures of Example 1(b) were repeated except that the product obtained in the above process (a) (2.40 g, 5.61 mmol), chloromethyl linolate (3.69 g, 11.2 mmol), anhydrous potassium carbonate (7 g) and sodium iodide (3.36 g, 22.4 mmol) were used to give 2',3',5'-O-tri-n-butyryl-3-linolyloxymethyl-5-fluorouridine as colorless caramel (3.45 g) (yield: 82.3 %). The solubility of this compound in soybean oil was >300 mg/soybean oil 1 ml.
   IR(Neat): νₘₐₓ (cm⁻¹) 1750, 1690, 1465, 1280, 1250, 1160, 1100
   FAB-MS (m/z): 787 (M+Na⁺)

### Example 20

### Preparation of the compound (I) wherein R¹=CH₃(CH₂)₅COO, R²=R³=R⁴=n-butyryloxy, R=H, Y=F

The procedures of Example 1(b) were repeated except that the product obtained in Example 19(a) (3.00 g, 7.01 mmol), chloromethyl n-heptanoate (2.51 g, 14.4 mmol), anhydrous potassium carbonate (8 g) and sodium iodide (4.21 g) were used to give 2',3',5'-O-tri-n-butyryl-3-n-heptanoyloxymethyl-5-fluorouridine as pale yellow caramel (3.41 g) (yield: 79.1 %). The solubility of this compound in soybean was >300 mg/soybean oil 1 ml.
IR(Neat): νₘₐₓ (cm⁻¹) 1750, 1690, 1465, 1280, 1160, 1100
FAB-MS (m/z): 615 (MH⁺)

### Example 21

### Preparation of the compound (I) wherein R¹=CH₃(CH₂)₅COO, R²=H, R³=R⁴=n-butyryloxy, R=H, Y=F

(a) The procedures of Example 19(a) were repeated except that 5-fluoro-5'-deoxyuridine (Flutone)(1.5 g, 6.1 mmol) and n-butyryl chloride (1.62 g, 2.5 equivalents) were used to give 2',3'-O-di-n-butyryl-5-fluoro-5'-deoxyuridine (2.03 g) (yield: 88.9 %).
   IR(Neat): νₘₐₓ (cm⁻¹) 3200, 3090, 2970, 1750, 1730, 1710, 1665
   FAB-MS (m/z): 387 (MH⁺)
(b) The procedures of Example 19(b) were repeated except that the product obtained in the above process (a) (1.43 g, 1.5 equivalents), chloromethyl n-heptanoate (1.43 g, 1.5 equivalents), anhydrous potassium carbonate (2.66 g, 3.6 equivalents) and sodium iodide (1.60 g, 2 equivalents) were used to give 2',3'-O-di-n-butyryl-3-n-heptanoyloxymethyl-5-fluoro-5'-deoxyuridine (2.42 g) (yield: 88.5 %).
   IR(Neat): νₘₐₓ (cm⁻¹) 2970, 2940, 1745, 1695, 1680
   FAB-MS (m/z): 529 (M+Na⁺)

### Example 22

### Preparation of the compound (I) wherein R¹=CH₃(CH₂)₆COO, R²=R³=CH₃CH₂COO, R⁴=H, R=H, Y=F

(a) The procedures of Example 1(a) were repeated except that 5-fluoro-2'-deoxyuridine (3 g, 12.2 mmol), anhydrous potassium carbonate (9.10 g), sodium iodide (6.58 g) and chloromethyl n-octanoate (4.70 g, 2 equivalents) were used to give 3-n-octanoyloxymethyl-2'-deoxy-5-fluorouridine as colorless crystal (3.17 g) (yield: 64.7 %) (melting point: 74.0 to 77.9°C).
(b) The procedures of Example 1(b) were repeated except that the product obtained in the above process (a) (1.50 g, 3.72 mmol), pyridine (30 ml) and propionyl chloride (1.03 g) were used to give 3',5'-dipropionyl-3-n-octanoyloxymethyl-2'-deoxy-5-fluorouridine as pale yellow oil (1.07 g) (yield: 55.8 %).
   IR(Neat): νₘₐₓ (cm⁻¹) 1740, 1680
   FAB-MS (m/z): 515 (MH⁺)

### Example 23

### Preparation of the compound (I) wherein R¹=CH₃(CH₂)₆COO, R²=R³=CH₃OCOO, R⁴=H, R=H, Y=F

The procedures of Example 1(b) were repeated except that the product obtained in Example 22(a) (2.01 g, 5 mmol) and methyl chlorocarbonate (9.4 g) were used to give 3',5'-O-dimethoxycarbonyl-3-n-octanoyloxymethyl-2'-deoxy-5-fluorouridine as colorless oil (1.71 g) (yield: 66.0 %).
IR(Neat): νₘₐₓ (cm⁻¹) 1750, 1680
FAB-MS (m/z): 519 (MH⁺)

### Example 24

### Preparation of the compound (I) wherein R¹=CH₃(CH₂)₅COO, R²=R³=i-BuO-COO, R⁴=H, R=H, Y=F

The procedures of Example 1(b) were repeated except that the product obtained in Example 4(a) (1.50 g, 3.86 mmol), i-butyl chloroformate (1.19 g) and pyridine (30 ml) were used to give 3',5'-O-di-i-butoxycarbonyl-3-n-heptanoyloxymethyl-2'-deoxy-5-fluorouridine as pale yellow oil (1.97 g) (yield: 86.5 %).
IR(Neat): νₘₐₓ (cm⁻¹) 3095, 2960, 2930, 2875, 1748, 1695, 1680
FAB-MS (m/z): 589 (MH⁺)

### Example 25

### Preparation of the compound (I) wherein R¹=CH₃(CH₂)₂COO, R²=R³=CH₃(CH₂)₆COO, R⁴=H, R=H, Y=F

(a) The procedures of Example 1(a) were repeated except that 5-fluoro-2'-deoxyuridine (2.2 g, 8.94 mmol), anhydrous potassium carbonate (6.67 g), sodium iodide (4.82 g) and chloromethyl n-butyrate (2.44 g, 2 equivalents) were used to give 3-n-butyryloxymethyl-2'-deoxy-5-fluorouridine as white powder (1.82 g) (yield: 58.9 %) (melting point: 103.0 to 105.0°C).
(b) The procedures of Example 1(b) were repeated except that the product obtained in the above process (a) (1.50 g, 4.33 mmol), pyridine (20 ml) and n-octanoyl chloride (1.76 g) were used to give 3',5'-di-n-octanoyl-3-n-butyryloxymethyl-2'-deoxy-5-fluorouridine as pale yellow oil (1.16 g) (yield: 44.6 %).
   IR(Neat): νₘₐₓ (cm⁻¹) 1740, 1681
   FAB-MS (m/z): 599 (MH⁺)

### Example 26

### Preparation of the compound (I) wherein R¹=CH₃(CH₂)₅COO, R²=R³=phenoxycarbonyloxy R⁴=H, R=H, Y=F

The procedures of Example 1(b) were repeated except that the product obtained in Example 4(a) (0.80 g, 2.06 mmol) and phenyl chloroformate (1.04 g, 6.64 mmol, 3.2 equivalents) were used to give 3',5'-O-diphenoxylcarbonyl-3-n-heptanoyloxymethyl-2'-deoxy-5-fluorouridine as pale yellow oil (1.22 g) (yield: 94.2 %).
IR(Neat): νₘₐₓ (cm⁻¹) 1760, 1740, 1695, 1680
FAB-MS (m/z): 629 (MH⁺)

### Example 27

### Preparation of the compound (I) wherein R¹=CH₃(CH₂)₅COO, R²=R³=n-butoxycarbonyloxy R⁴=H, R=H, Y=CF₃

(a) The procedures of Example 1(a) were repeated except that 5-trifluoromethyl-2'-deoxyuridine (1.70 g, 5.74 mmol), anhydrous potassium carbonate (4.73 g), sodium iodide (2.56 g) and chloromethyl n-heptanoate (2.50 g, 2.4 equivalents) were used to give 3-n-heptanoyloxymethyl-2'-deoxy-5-trifluoromethyluridine as pale yellow oil (1.83 g) (yield: 71.3 %).
   IR(Neat): νₘₐₓ (cm⁻¹) 3500, 2960, 2920, 2860, 1740, 1680
   FAB-MS (m/z): 461 (M+Na), 439 (MH⁺)
(b) The procedures of Example 1(b) were repeated except that the product obtained in the above process (a) (1.65 g, 3.76 mmol), pyridine (20 ml) and n-butyl chloroformate (2.5 g, 23.5 mmol) were used to give 3',5'-di-n-butoxycarbonyl-3-n-heptanoyloxymethyl-2'-deoxy-5-trifluoromethyluridine as colorless crystal (2.11 g) (yield: 77.4 %) (melting point: 90 to 91°C).
   IR(Nujol): νₘₐₓ (cm⁻¹) 1755, 1680
   FAB-MS (m/z): 639 (MH⁺)

### Example 28

### Preparation of the compound (I) wherein R¹=CH₃(CH₂)₅COO, R²=R³=n-butyryloxy, R⁴=H, R=H, Y=CF₃

The procedures of Example 1(b) were repeated except that the product obtained in Example 27(a) (1.50 g, 3.42 mmol), triethylamine (1.73 g, 17.1 mmol) and butyryl chloride (1.09 g, 10.3 mmol) were used to give 3',5'-O-di-n-butyryl-3-n-heptanoyloxymethyl-2'-deoxy-5-trifluoromethyluridine as pale yellow oil (1.76 g) (yield: 88.9 %).
IR(Neat): νₘₐₓ (cm⁻¹) 2960, 2920, 2860, 1740, 1690
FAB-MS (m/z): 579 (MH⁺)

### Example 29

### Preparation of the compound (I) wherein R¹=CH₃(CH₂)₅COO, R²=R³=n-butyryloxy, R⁴=H, R=H, Y=F

(a) The procedures of Example 1(a) were repeated except that 5-fluoro-2'-deoxyuridine (960 mg, 3.9 mmol), anhydrous potassium carbonate (3.23 g), sodium iodide (2.30 g) and 1-chloroethyl n-heptanoate (1.50 g, 2 equivalents) were used to give 3-n-heptanoyloxyethyl-2'-deoxy-5-fluorouridine as pale yellow oil (557 mg).
   IR(Neat): νₘₐₓ (cm⁻¹) 3480, 2930, 1725, 1660
   FAB-MS (m/z): 403 (MH⁺)
(b) The procedures of Example 1(b) were repeated except that the product obtained in the above process (a) (520 mg, 1.29 mmol), pyridine (7 ml) and butyryl chloride (412 mg, 3.87 mmol) were used to give 3',5'-O-di-n-butyryl-3-n-heptanoyloxyethyl-2'-deoxy-5-fluorouridine as pale yellow oil (623 mg) (yield: 89.0 %).
   IR(Nujol): νₘₐₓ (cm⁻¹) 2970, 2930, 1750, 1680
   FAB-MS (m/z): 543 (MH⁺)
   The compounds obtained in Examples 1 to 29 are summarized in the following Table 1.

**Table 1**

| Ex. | R¹ | R²,R³ | R⁴ | R | Y |
|---|---|---|---|---|---|
| 1 | Linolyloxy | CH₃(CH₂)₅COO | H | H | F |
| 2 | Linolyloxy | n-BuOCO(CH₂)₂COO | H | H | F |
| 3 | Linolyloxy | CH₃COO | H | H | F |
| 4 | CH₃(CH₂)₅COO | CH₃(CH₂)₂COO | H | H | F |
| 5 | CH₃(CH₂)₅COO | (CH₃)₂CHCOO | H | H | F |
| 6 | CH₃(CH₂)₅COO | (CH₃CH₂CH₂)₂CHCOO | H | H | F |
| 7 | CH₃(CH₂)₅COO | CH₃(CH₂)₅COO | H | H | F |
| 8 | CH₃(CH₂)₅COO | n-BuOCO(CH₂)₂COO | H | H | F |
| 9 | CH₃(CH₂)₅COO | n-BuOCO(CH₂)₃COO | H | H | F |
| 10 | CH₃(CH₂)₁₄COO | (CH₃)₂CHCOO | H | H | F |
| 11 | CH₃(CH₂)₁₄COO | CH₃(CH₂)₅COO | H | H | F |
| 12 | CH₃(CH₂)₁₄COO | n-BuOCO(CH₂)₂COO | H | H | F |
| 13 | n-BuOCO(CH₂)₃COO | CH₃(CH₂)₅COO | H | H | F |
| 14 | t-BuCOO | CH₃(CH₂)₅COO | H | H | F |
| 15 | Linolyloxy | Phenoxycarbonyloxy | H | H | F |
| 16 | Linolyloxy | 4-methoxyphenoxycarbonyloxy | H | H | F |
| 17 | CH₃(CH₂)₅COO | n-BuOCOO | H | H | F |
| 18 | Linolyloxy | CH₃(CH₂)₂COO(R²) 4-Chlorobenzyloxy(R³) | H | H | F |
| 19 | Linolyloxy | CH₃(CH₂)₂COO | CH₃(CH₂)₂COO | H | F |
| 20 | CH₃(CH₂)₅COO | CH₃(CH₂)₂COO | CH₃(CH₂)₂COO | H | F |
| 21 | CH₃(CH₂)₅COO | H(R²) CH₃(CH₂)₂COO(R³) | CH₃(CH₂)₂COO | H | F |
| 22 | CH₃(CH₂)₆COO | CH₃CH₂COO | H | H | F |
| 23 | CH₃(CH₂)₆COO | CH₃OCO | H | H | F |
| 24 | CH₃(CH₂)₅COO | iBu-OCOO | H | H | F |
| 25 | CH₃(CH₂)₂COO | CH₃(CH₂)₆COO | H | H | F |
| 26 | CH₃(CH₂)₅COO | Phenoxycarbonyloxy | H | H | F |
| 27 | CH₃(CH₂)₅COO | n-BuOCOO | H | H | CF₃ |
| 28 | CH₃(CH₂)₅COO | CH₃(CH₂)₂COO | H | H | CF₃ |
| 29 | CH₃(CH₂)₅COO | CH₃(CH₂)₂COO | H | CH₃ | F |

### Preparation

The fluorouridine derivative (4.5 g) obtained in Example 4 and purified soybean oil (30 g) were mixed together with heating at 40 to 70°C and thereto was added purified yolk lecithin (14.4 g) which was previously dispersed in water (150 ml). This mixture was stirred with homogenizer (ULTRA-TURRAX TP 18-10) at 10,000 rpm for about 10 minutes to give a crude emulsion. Three hundred milliliters of the crude emulsion was taken and pH was adjusted to 7 with 1N-NaOH etc. and finely emulsified under high pressure (1,000 kg/cm²) with nanomizer system (LA-11) to give particulate lipid microspheres with average particle size of 105 nm.

## Claims

1. A uridine derivative having the formula (I): 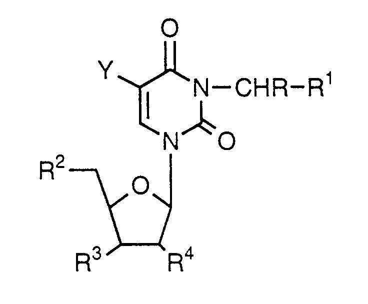 wherein R¹ is acyloxy; R² is hydrogen, phenoxycarbonyloxy (optionally substituted by a lower alkoxy), alkoxycarbonyloxy or acyloxy; R³ is aralkyloxy, phenoxycarbonyloxy (optionally substituted by a lower alkoxy), alkoxycarbonyloxy or acyloxy; R⁴ is hydrogen, phenoxycarbonyloxy (optionally substituted by a lower alkoxy), alkoxycarbonyloxy or acyloxy; R is hydrogen or a lower alkyl; and Y is fluorine or trifluoromethyl.

2. A compound as claimed in Claim 1 in which R¹ is a saturated or unsaturated aliphatic carbonyloxy having 4 to 20 carbon atoms or an alkanoyl having 2 to 6 carbon atoms substituted by an alkoxycarbonyl having 2 to 7 carbon atoms, R² is hydrogen, phenoxycarbonyloxy (optionally substituted by an alkoxy having 1 to 6 carbon atoms), an alkoxycarbonyloxy having 2 to 20 carbon atoms, a straight chain or branched chain alkanoyl having 2 to 10 carbon atoms or an alkanoyl having 2 to 6 carbon atoms substituted by an alkoxycarbonyl having 2 to 7 carbon atoms, R³ is an aralkyloxy, phenoxycarbonyloxy (optionally substituted by an alkoxy having 1 to 6 carbon atoms), an alkoxycarbonyloxy having 2 to 20 carbon atoms, a straight chain or branched chain alkanoyl having 2 to 10 carbon atoms or alkanoyl having 2 to 6 carbon atoms substituted by an alkoxycarbonyl having 2 to 7 carbon atoms, R⁴ is hydrogen, phenoxycarbonyloxy (optionally substituted by an alkoxy having 1 to 6 carbon atoms), an alkoxycarbonyloxy having 2 to 20 carbon atoms, a straight chain or branched chain alkanoyl having 2 to 10 carbon atoms or an alkanoyl having 2 to 6 carbon atoms substituted by an alkoxycarbonyl having 2 to 7 carbon atoms, R is hydrogen or alkyl having 1 to 6 carbon atoms.

3. A compound as claimed in Claim 2 in which R¹ is heptanoyloxy, R² and R³ are butyryloxy or n-butoxycarbonyloxy, R⁴ is hydrogen, R is hydrogen, Y is fluorine.

4. A pharmaceutical composition comprising an effective amount of the compound as claimed in Claim 1 or a pharmaceutically acceptable salt thereof and pharmaceutically acceptable carrier, diluent or disintegrant.

5. A process for preparing a uridine derivative having the formula (I): 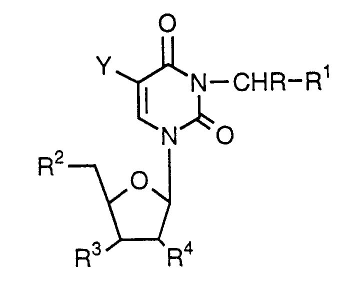 wherein R¹ is acyloxy; R² is hydrogen, phenoxycarbonyloxy (optionally substituted by a lower alkoxy), alkoxycarbonyloxy or acyloxy; R³ is aralkyloxy, phenoxycarbonyloxy (optionally substituted by a lower alkoxy), alkoxycarbonyloxy or acyloxy; R⁴ is hydrogen, phenoxycarbonyloxy (optionally substituted by a lower alkoxy), alkoxycarbonyloxy or acyloxy; R is hydrogen or a lower alkyl; and Y is fluorine or trifluoromethyl, which comprises reacting a uridine derivative having the formula (II): 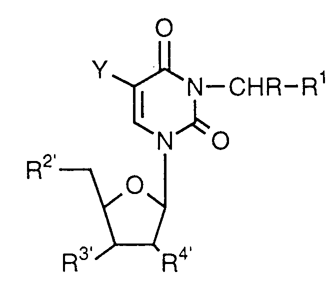 wherein R¹, R and Y are as defined above; R^{2'} is hydrogen or hydroxy; R^{3'} is aralkyloxy or hydroxy; and R^{4'} is hydrogen or hydroxy, with a compound having the formula (III):
R⁵OH
wherein R⁵ is phenoxycarbonyl (optionally substituted by a lower alkoxy), alkoxycarbonyl or acyl, or a reactive derivative thereof.

6. A process for preparing a uridine derivative having the formula (I): 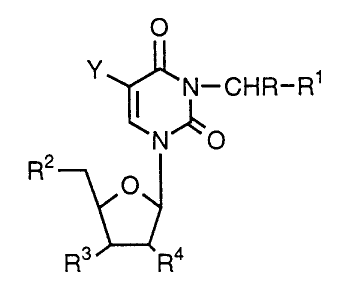 wherein R¹ is acyloxy; R² is hydrogen, phenoxycarbonyloxy (optionally substituted by a lower alkoxy), alkoxycarbonyloxy or acyloxy; R³ is aralkyloxy, phenoxycarbonyloxy (optionally substituted by a lower alkoxy), alkoxycarbonyloxy or acyloxy; R⁴ is hydrogen, phenoxycarbonyloxy (optionally substituted by a lower alkoxy), alkoxycarbonyloxy or acyloxy; R is hydrogen or a lower alkyl; and Y is fluorine or trifluoromethyl, which comprises reacting a uridine derivative having the formula (IV): 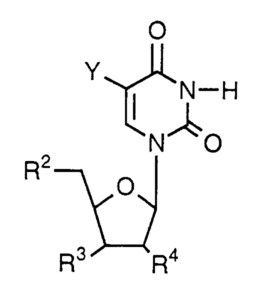 wherein R², R³, R⁴ and Y are as defined above, with a compound having the formula (V):
R¹RCHX
wherein R¹ and R are as defined above, and X is a reactive residue.

7. Use of the compound as claimed in Claim 1 for preparing an anti-tumor agent.
